# NEW EUROPEAN PATENT SPECIFICATION

(11) **EP 1 173 177 B2**
(45) Date of publication and mention of the opposition decision: **05.03.2014**
(45) Mention of the grant of the patent: 03.11.2004
(21) Application number: 00921047.7
(22) Date of filing: 26.04.2000
(51) Int. Cl.: A61K 31/436, A61P 27/02

(54) **USE OF MACROLIDE COMPOUNDS FOR THE TREATMENT OF DRY EYE**
VERWENDUNG VON MAKROLIDEN ZUR BEHANDLUNG VON TROCKENEN AUGEN
UTILISATION DE COMPOSES MACROLIDES POUR LE TRAITEMENT DES YEUX SECS

(30) Priority: 30.04.1999 US 132009 P
(43) Date of publication of application: 23.01.2002
(73) Proprietor: Sucampo AG, 6300 Zug (CH)
(72) Inventor: UENO, Ryuji, Montgomery, MD 20854 (US)
(74) Representative: Gille Hrabal
(86) International application number: PCT/JP2000/002756
(87) International publication number: WO 2000/066122

(56) References cited:
- EP-A- 0 532 862
- WO-A-00/09109
- WO-A-96/31514
- WO-A-97/25977
- YANG, JIHONG ET AL: "Sjogren's syndrome in mice carrying the lprcg gene and the therapeutic efficacy of an immunosuppressive agent FK506" PATHOL. INT. (1999), 49(2), 133-140 , - February 1999 (1999-02) XP000952466
- DATABASE WPI Derwent Publications Ltd., London, GB; AN 2000-038597 XP002150034 YAMANAKA MASAYUKI: "Compositions containing macrolide compounds have high stability and adsorbability." & WO 99 55332 A (FUJISAWA PHARMA CO LTD), 16 November 1999 (1999-11-16)
- TSUBOTA K: "New approaches to dry-eye therapy" INTERNATIONAL OPHTHALMOLOGY CLINICS,US,LITTLE, BROWN, BOSTON, vol. 34, no. 1, 1994, pages 115-128, XP002120709 ISSN: 0020-8167
- IWAMOTO H ET AL: "Inhibitory effects of FK506 on the development of experimental allergic/immune-mediated blepharoconjunctivitis in Lewis rats by systemic but not by topical administration." GRAEFES ARCHIVE FOR CLINICAL AND EXPERIMENTAL OPHTHALMOLOGY, (1999 MAY) 237 (5) 407-14. , XP000952455
- TSUJIKAWA A ET AL: "TACROLIMUS (FK506) ATTENUATES LEUKOCYTE ACCUMULATION AFTER TRANSIENT RETINAL ISCHEMIA" STROKE,US,AMERICAN HEART ASSOCIATION, DALLAS TX, vol. 29, no. 7, 1998, pages 1431-1438, XP000913599 ISSN: 0039-2499
- TABBARA K F ET AL: "DRY EYE SYNDROME" DRUGS OF TODAY / MEDICAMENTOS DE ACTUALIDAD,ES,J.R. PROUS SS.A. INTERNATIONAL PUBLISHERS, vol. 34, no. 5, 1998, pages 447-453, XP000872457 ISSN: 0025-7656

## Description

### Technical Field

The present invention relates to an agent for treating a dry eye.

### Background Art

One of the symptoms of ophthalmic diseases drawing much attention these days is dry eye. The dry eye is defined to mean a condition wherein lacrimal fluid is less in amount or abnormal in quality, with or without the presence of corneal and conjunctival lesion (Yamada, M. et al., Folia Ophthalmol. Jpn., 43, 1289-1293 (1992)). Specific symptoms include dry eye observed in hypolacrimation, alacrima, xerophthalmia, Sjögren syndrome, keratoconjunctivitis sicca, Stevens-Johnson syndrome, ocular pemphigoid, marginal blepharitis, diabetes and the like, dry eye observed after cataract operation, dry eye in conjunction with allergic conjunctivitis and the like, and dry eye due to hypolacrimation caused by increased VDT (visual display terminal) work, dry room with air conditioning and the like.

The dry eye is caused by various factors that may not be entirely clear, and, at the moment, a drastic treatment method, such as promotion of the secretion of lacrimal fluid, has not been established yet. Therefore, the dry eye has been diagnosed according to the subjective symptoms obtained by questioning and objective symptoms known from lacrimal fluid evaluation tests (tear film breakup time, Schirmer test, lacrimal fluid clearance test and the like), corneal and conjunctival staining tests (fluorescein staining, rose bengale staining and the like), and the like. For example, tear film breakup time (BUT), which is one of the lacrimal fluid evaluation tests, reflects the stability of precorneal tear film, and means the time (sec) from complete nictitation to the initial breakage of the precorneal tear film. A lower BUT means severer dry eye symptom. In the case of severe dry eye, the breakage of the tear film occurs immediately after nictitation, which is rated as BUT zero (0) sec.

At present, a dry eye therapy includes increasing lacrimal fluid reservoir in conjunctival sac by instillation of artificial tears to alleviate the subjective symptoms of patients or to protect the eye from drying, and other methods.

For the above-mentioned therapy, instillation of chondroitin sulfate, methyl cellulose and the like, and internal use of bromhexine hydrochloride, salivary gland hormone and the like have been the typical methods. However, the effect of such therapy is not necessarily satisfactory. While instillation of artificial tears and use of a goggle eye patch and the like have been the means to protect the eyes from drying, these are not more than auxiliary therapy methods.

### DISCLOSURE OF THE INVENTION

As a result of the intensive studies done by the present inventor, it was surprisingly found that a macrolide compound has a superior improving effect on dry eye symptoms, particularly subjective symptoms, and in lacrimal fluid evaluation tests, such as tear film breakup time and the like, and exhibits a superior therapeutic effect on the dry eye, which resulted in the completion of the present invention.

Accordingly, the present invention provides the following.
(1)The use of a macrolide compound for the manufacture of a pharmaceutical agent for the improvement of the tear film breakup time by local administration to the eye, wherein the macrolide compound is a tricyclo compound (I) of the following formula 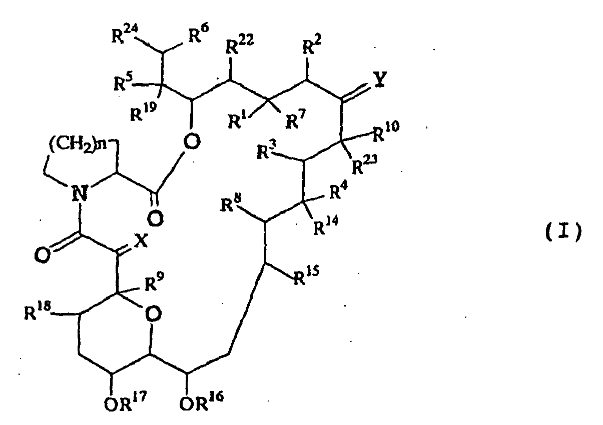 wherein adjacent pairs of R¹ and R², R³ and R⁴, and R⁵ and R⁶ each independently
   a) consist of two adjacent hydrogen atoms, wherein R² is optionally alkyl, or
   b) form another bond between carbon atoms binding with the members of each pair;
   R⁷ is hydrogen atom, hydroxy, alkyloxy or protected hydroxy, or may form oxo with R¹;
   R⁸ and R⁹ each independently show hydrogen atom or hydroxy;
   R¹⁰ is hydrogen atom, alkyl, alkenyl, alkyl substituted by one or more hydroxy, alkenyl substituted by one or more hydroxy, or alkyl substituted by oxo;
   X is oxo, (hydrogen atom, hydroxy), (hydrogen atom, hydrogen atom), or a group of the formula -CH₂O-;
   Y is oxo, (hydrogen atom, hydroxy), (hydrogen atom, hydrogen atom), or a group of the formula N-NR¹¹R¹² or N-OR¹³;
   R¹¹ and R¹² each independently show hydrogen atom, alkyl, aryl or tosyl; R¹³, R¹⁴, R¹⁵, R¹⁶, R¹⁷, R¹⁸, R¹⁹, R²² and R²³ each independently show hydrogen atom or alkyl;
   R²⁴ is an optionally substituted ring that may contain one or more hetero atom(s);and
   n is 1 or 2.
   In addition to the meaning noted above, Y, R¹⁰ and R²³ may form, together with the carbon atom they bind with, a saturated or unsaturated 5 or 6-membered heterocyclic group containing nitrogen atom, sulfur atom and/or oxygen atom, wherein the heterocyclic group may be substituted by one or more group(s) selected from the group consisting of alkyl, hydroxy, alkyloxy, benzyl, a group of the formula -CH₂Se(C₆H₅), and alkyl substituted by one or more hydroxy, or a pharmaceutically acceptable salt thereof.
(2) The use of (1), wherein the macrolide compound is FK506.

### DETAILED DESCRIPTION OF THE INVENTION

Some of themacrolide compounds to be used in the present invention are known as shown below and a novel macrolide compound can be prepared from these known macrolide compounds by a known method. Preferable examples thereof include macrolide compounds such as FK506, Ascomycin derivative, Rapamycin derivative and the like.

The macrolide compounds of the present invention are tricyclo compounds (I) of the following formula and a pharmaceutically acceptable salt thereof. 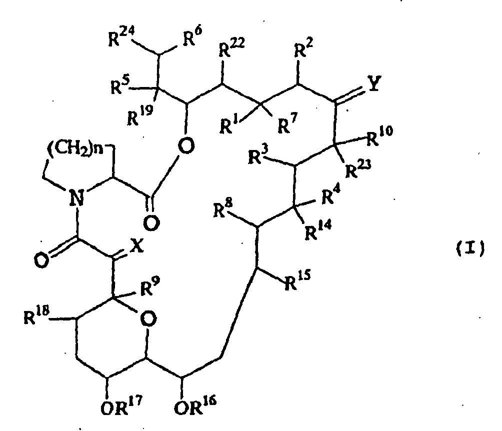 wherein adjacent pairs of R¹ and R², R³ and R⁴, and R⁵ and R⁶ each independently
a) consist of two adjacent hydrogen atoms, wherein R² is optionally alkyl, or
b) form another bond between carbon atoms binding with the members of each pair;
R⁷ is hydrogen atom, hydroxy, alkyloxy or protected hydroxy, or may form oxo with R¹;
R⁸ and R⁹ each independently show hydrogen atom or hydroxy;
R¹⁰ is hydrogen atom, alkyl, alkenyl, alkyl substituted by one or more hydroxy, alkenyl substituted by one or more hydroxy, or alkyl substituted by oxo;
X is oxo, (hydrogen atom, hydroxy), (hydrogen atom, hydrogen atom), or a group of the formula -CH₂O-;
Y is oxo, (hydrogen atom, hydroxy), (hydrogen atom, hydrogen atom), or a group of the formula N-NR¹¹R¹² or N-OR¹³;
R¹¹ and R¹² each independently show hydrogen atom, alkyl, aryl or tosyl;
R¹³, R¹⁴, R¹⁵, R¹⁶, R¹⁷, R¹⁸, R¹⁹, R²² and R²³ each independently show hydrogen atom or alkyl;
R²⁴ is an optionally substituted ring that may contain one or more hetero atom(s); and
n is 1 or 2.

In addition to the meaning noted above, Y, R¹⁰ and R²³ may form, together with the carbon atom they bind with, a saturated or unsaturated 5 or 6-membered heterocyclic group containing nitrogen atom, sulfur atom and/or oxygen atom, wherein the heterocyclic group may be substituted by one or more group(s) selected from the group consisting of alkyl, hydroxy, alkyloxy, benzyl, a group of the formula -CH₂Se(C₆H₅), and alkyl substituted by one or more hydroxy.

Preferable R²⁴ is, for example, cyclo(C₅-C₇)alkyl optionally having suitable substituent, such as the following.
(a) 3,4-dioxocyclohexyl,
(b) 3-R²⁰-4-R²¹-cyclohexyl,
   wherein R²⁰ is hydroxy, alkyloxy or -OCH₂OCH₂CH₂OCH₃, and
   R²¹ is hydroxy, -OCN, alkyloxy, heteroaryloxy optionally having suitable substituent, -OCH₂OCH₂CH₂OCH₃, protected hydroxy, chloro, bromo, iodo, aminooxalyloxy, azide, p-tolyloxythiocarbonyloxy, or R²⁵R²⁶CHCOO- (wherein R²⁵ is hydroxy optionally protected where desired or protected amino, and R²⁶ is hydrogen atom or methyl),
   or R²⁰ and R²¹ in combination form an oxygen atom of epoxide ring, and
(c) cyclopentyl substituted by methoxymethyl, protected hydroxymethyl where desired, acyloxymethyl (wherein acyl moiety is optionally quaternized dimethylamino where desired or optionally esterified carboxy), one or more optionally protected amino and/or hydroxy, or aminooxalyloxymethyl. Preferable example includes 2-formyl-cyclopentyl.

The definition of each symbol used in the formula (I), specific examples thereof and preferable embodiments thereof are explained in detail in the following.

"Lower" means that a group has 1 to 6 carbon atoms unless otherwise indicated.

Preferable examples of "alkyl" and the alkyl moiety of "alkyloxy" include linear or branched aliphatic hydrocarbon residue, such as lower alkyl (e.g., methyl, ethyl, propyl, isopropyl, butyl, isobutyl, pentyl, neopentyl, hexyl and the like).

Preferable examples of "alkenyl" include linear or branched aliphatic hydrocarbon residue having one double bond, such as lower alkenyl (e.g., vinyl, propenyl (e.g., allyl and the like), butenyl, methylpropenyl, pentenyl, hexenyl and the like).

Preferable examples of "aryl" include phenyl, tolyl, xylyl, cumenyl, mesityl, naphthyl and the like.

Preferable examples of the protective group of "protected hydroxy" and "protected amino" include 1-(lower alkylthio)(lower)alkyl such as lower alkylthiomethyl (e.g., methylthiomethyl, ethylthiomethyl, propylthiomethyl, isopropylthiomethyl, butylthiomethyl, isobutylthiomethyl, hexylthiomethyl and the like), with more preference given to C₁ - C₄ alkylthiomethyl and most preference given to methylthiomethyl;
tri-substituted silyl such as tri(lower)alkylsilyl (e.g., trimethylsilyl, triethylsilyl, tributylsilyl, tert-butyldimethylsilyl, tri-tert-butylsilyl and the like), and lower alkyldiarylsilyl (e.g., methyldiphenylsilyl, ethyldiphenylsilyl, propyldiphenylsilyl, tert-butyldiphenylsilyl and the like, with more preference given to tri(C₁-C₄)alkylsilyl and C₁-C₄ alkyldiphenylsilyl, and most preference given to tert-butyldimethylsilyl, tert-butyldiphenylsilyl;
acyl such as aliphatic acyl derived from carboxylic acid, sulfonic acid and carbamic acid, aromatic acyl, and aliphatic acyl substituted by aromatic group; and the like.

The aliphatic acyl is exemplified by lower alkanoyl optionally having one or more suitable substituent(s) (e.g., carboxy) such as formyl, acetyl, propionyl, butyryl, isobutyryl, valeryl, isovaleryl, pivaloyl, hexanoyl, carboxyacetyl, carboxypropionyl, carboxybutyryl, carboxyhexanoyl and the like;
cyclo(lower)alkyloxy(lower)alkanoyl optionally having one or more suitable substituent(s) (e.g., lower alkyl) such as cyclopropyloxyacetyl, cyclobutyloxypropionyl, cycloheptyloxybutyryl, mentyloxyacetyl, mentyloxypropionyl, mentyloxybutyryl, mentyloxypentanoyl, mentyloxyhexanoyl and the like, camphorsulfonyl; lower alkylcarbamoyl having one or more suitable substituent(s) such as carboxy or protected carboxy and the like, such as carboxy(lower)alkylcarbamoyl (e.g., carboxymethylcarbamoyl, carboxyethylcarbamoyl, carboxypropylcarbamoyl, carboxybutylcarbamoyl, carboxypentylcarbamoyl, carboxyhexylcarbamoyl) and
tri(lower)alkylsilyl(lower)alkyloxycarbonyl(lower)-alkylcar bamoyl (e.g., trimethylsilylmethoxycarbonylethylcarbamoyl, trimethylsilylethoxycarbonylpropylcarbamoyl, triethylsilylethoxycarbonylpropylcarbamoyl, tert-butyldimethylsilylethoxycarbonylpropylcarbamoyl, trimethylsilylpropoxycarbonylbutylcarbamoyl); and the like.

Aromatic acyl is exemplified by aroyl optionally having suitable substituent(s) (e.g., nitro), such as benzoyl, toluoyl, xyloyl, naphthoyl, nitrobenzoyl, dinitrobenzoyl, nitronaphthoyl and the like; and arenesulfonyl optionally having one or more suitable substituent (s) (e.g., halogen), such as benzenesulfonyl, toluenesulfonyl, xylenesulfonyl, naphthalenesulfonyl, fluorobenzenesulfonyl, chlorobenzenesulfonyl, bromobenzenesulfonyl, iodobenzenesulfonyl and the like.

The aliphatic acyl substituted by aromatic group may be, for example, ar(lower)alkanoyl optionally having one or more suitable substituent(s) (e.g., lower alkyloxy or trihalo(lower)alkyl and the like), wherein specific examples are phenylacetyl, phenylpropionyl, phenylbutyryl, 2-trifluoromethyl-2-methoxy-2-phenylacetyl, 2-ethyl-2-trifluoromethyl-2-phenylacetyl, 2-trifluoromethyl-2-propoxy-2-phenylacetyl and the like.

Of the above-mentiond acyl, more preferable acyl includes C₁ - C₄ alkanoyl optionally having carboxy, cyclo(C₅ - C₆)alkyloxy(C₁ - C₄)alkanoyl having two (C₁ - C₄)alkyl in the cycloalkyl moiety, camphorsulfonyl, carboxy (C₁ - C₄)alkylcarbamoyl, tri(C₁ - C₄)alkylsilyl(C₁ - C₄)alkyloxycarbonyl(C₁ - C₄)alkylcarbamoyl, benzoyl optionally having 1 or 2 nitro groups, benzenesulfonyl having halogen, and phenyl(C₁ - C₄)alkanoyl having C₁ - C₄ alkyloxy and trihalo(C₁ - C₄)alkyl. Of these, most preferred are acetyl, carboxypropionyl, mentyloxyacetyl, camphorsulfonyl, benzoyl, nitrobenzoyl, dinitrobenzoyl, iodobenzenesulfonyl, 2-trifluoromethyl-2-methoxy-2-phenylacetyl and the like.

Preferable examples of the "heterocyclic group consisting of saturated or unsaturated 5 or 6-membered ring having nitrogen atom, sulfur atom and/or oxygen atom" are pyrolyl, tetrahydrofuryl and the like.

The "heteroaryl optionally having a suitable substituent" moiety of the "heteroaryloxy optionally having a suitable substituent" is that exemplified for R¹ of the compound of the formula I of EP-A-532,088, with preference given to 1-hydroxyethylindol-5-yl. This publication is incorporated hereinto by reference.

The tricyclo compound (I) and a pharmaceutically acceptable salt thereof to be used in the present invention have immunosuppressive action, antibacterial action and other pharmacological activity, so that they are useful for the prophylaxis and treatment of rejection in organ or tissue transplantation, graft versus host reaction, autoimmune diseases, infectious diseases and the like, as noted, together with the production method thereof, in, for example, EP-A-184162, EP-A-323042, EP-A-423714, EP-A-427680, EP-A-465426, EP-A-480623, EP-A-532088, EP-A-532089, EP-A-569337, EP-A-626385, WO89/05303, WO93/05058, WO96/31514, WO91/13889, WO91/19495, WO93/5059 and the like.

In particular, the compounds called FR900506 (=FK506), FR900520 (Ascomycin), FR900523 and FR900525 are produced by the genus Streptomyces, such as *Streptomyces tsukubaensis*, No. 9993 (depository: National Institute of Bioscience and Human-Technology Agency of Industrial Science and Technology, the Ministry of International Trade and Industry, 1-3, Higashi 1-chome, Tsukuba-shi, Ibaraki-ken, Japan (formerly: Fermentation Research Institute, Agency of Industrial Science and Technology, the Ministry of International Trade and Industry), date of deposit: October 5, 1984, deposit number: FERMBP-927) or *Streptomyces hygroscopicus subsp. Yakushimaensis,* No. 7238 (depository:National Institute of Bioscience and Human-Technology Agency of Industrial Science and Technology, 1-3, Higashi 1-chome, Tsukuba-shi, Ibaraki-ken, Japan (formerly:Fermentation Research Institute, Agency of Industrial Science and Technology, the Ministry of International Trade and Industry), date of deposit: January 12, 1985, deposit number:FERM BP-928 (EP-A-0184162)). The compound of the following formula, FK506 (general name: Tacrolimus), is a representative compound.

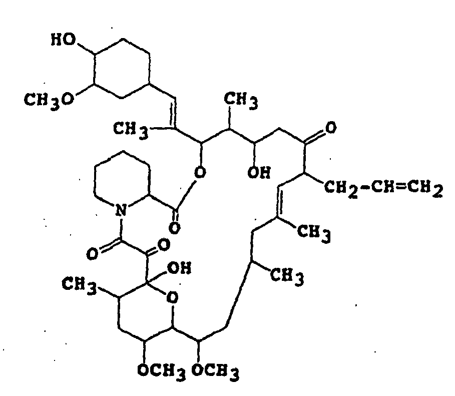

Chemical name:17-allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo-[22.3.1.0^{4,9}]octacos-18-ene-2,3,10,16-tetraone

Of the tricyclo compounds (I), more preferred is a compound wherein adjacent pairs of R³ and R⁴, and R⁵ and R⁶ each independently form another bond between carbon atoms binding with the members of each pair;
R⁸ and R²³ each independently show hydrogen atom;
R⁹ is hydroxy;
R¹⁰ is methyl, ethyl, propyl or allyl;
X is (hydrogen atom, hydrogen atom) or oxo;
Y is oxo;
R¹⁴, R¹⁵, R¹⁶, R¹⁷, R¹⁸, R¹⁹ and R²² each independently show methyl;
R²⁴ is 3-R²⁰-4-R²¹-cyclohexyl,
wherein R²⁰ is hydroxy, alkyloxy or -OCH₂OCH₂CH₂OCH₃, and
R²¹ is hydroxy, -OCN, alkyloxy, heteroaryloxy optionally having suitable substituent, -OCH₂OCH₂CH₂OCH₃, protected hydroxy, chloro, bromo, iodo, aminooxalyloxy, azide, p-tolyloxythiocarbonyloxy or R²⁵R²⁶CHCOO- (wherein R²⁵ is hydroxy optionally protected where desired, or protected amino, and R²⁶ is hydrogen atom or methyl), or R²⁰ and R²¹ in combination form an oxygen atom of epoxide ring; and n is 1 or 2.

Particularly preferable tricyclo compound (I) includes, besides FK506, Ascomycin derivatives such as halogenated derivative of 33-epi-chloro-33-desoxy Ascomycin described in Example 66a of EP-A-427,680 and the like.

Other preferable macrolide compounds include Rapamycin described in MERCK INDEX, 12 edition, No. 8288 and derivatives thereof. Preferable examples thereof include O-substituted derivative described at page 1 of WO95/16691, formula A, wherein the 40^{th} hydroxy is -OR₁ (wherein R₁ is hydroxyalkyl, hydroalkyloxyalkyl, acylaminoalkyl or aminoalkyl), such as 40-O-(2-hydroxy)ethyl Rapamycin, 40-O-(3-hydroxy)propyl Rapamycin, 40-O-[2-(2-hydroxy)ethoxy]ethyl Rapamycin and 40-O-(2-acetaminoethyl) Rapamycin. These O-substituted derivatives can be produced by reacting, under appropriate conditions, Rapamycin (or dihydro or deoxo Rapamycin) and an organic radical bound with a leaving group (e.g., RX wherein R is an organic radical desirable as O-substituent, such as alkyl, allyl and benzyl moiety, and X is a leaving group such as CCl₃C(NH)O and CF₃SO₃)). The conditions are: when X is CCl₃C(NH)O, acidic or neutral conditions, such as in the presence of trifluoromethanesulfonic acid, camphorsulfonic acid, p-toluenesulfonic acid or their corresponding pyridiniumor substituted pyridinium salt, and when X is CF₃SO₃, in the presence of a base such as pyridine, substituted pyridine, diisopropylethylamine and pentamethylpiperidine. The most preferable Rapamycin derivative is 40-O-(2-hydroxy)ethyl Rapamycin as disclosed in WO94/09010. The contents of the above references are hereby incorporated into the specification by reference.

The pharmaceutically acceptable salt of tricyclo compound (I), Rapamycin and derivatives thereof are nontoxic and pharmaceutically acceptable conventional salts, which are exemplified by salts with inorganic or organic base such as alkali metal salt (e.g., sodium salt, potassium salt and the like), alkaline earth metal salt (e.g., calcium salt, magnesium salt and the like) , ammonium salt, and amine salt (e.g., triethylamine salt, N-benzyl-N-methylamine salt and the like).

In the macrolide compound of the present invention, conformer or one or more pairs of stereoisomers, such as optical isomers and geometric isomers, may be included due to asymmetric carbon atom and double bond. Such conformers and isomers are also encompassed in the present invention. In addition, macrolide compounds can form solvates, which case is also encompassed in the present invention. Preferable solvate is exemplified by hydrates and ethanolates.

The diseases associated with dry eye include those mentioned above inclusive of hypolacrimation, alacrima xerophthalmia, Sjo̊gren syndrome, keratoconjunctivitis sicca, Stevens-Johnson syndrome, ocular pemphigoid, marginal blepharitis, diabetes and the like, dry eye observed after cataract operation, that in conjunction with allergic conjunctivitis and the like. The dry eye similar to hypolacrimatioin is also observed, which is caused by VDT work and dry room due to air conditioning and the like.

The treatment agent of the present invention is effective for the improvement of the tear film breakup time.

The macrolide compound to be used in the present invention can be used as a pharmaceutical agent for human and animals, and is administered locally, to a local site in the eye (inclusive of eye ointment).

The dose of the macrolide compound varies depending on the kind, age, body weight of the administration subject such as human and animal, conditions to be treated, desired therapeutic effect, administration method, treatment period and the like. When it is administered locally to the eye, a preparation containing the active ingredient in a proportion of 0.001 - 10.0 w/v%, preferably 0.005 - 5.0 w/v%, is applied to one eye several times a day, preferably instilled or applied 1 to 6 times a day.

According to the present invention, a macrolide compound, which is an active ingredient, can be administered alone or in combination with other pharmacologically active components. When administered after formulating a preparation, it can be administered as a preparation produced by a conventional method. The dosage formmay be, for example, eye drop or eye ointment. Such preparation can be produced according to a conventional method. When an eye drop is desired, an eye drop as described in EP-A-0406791 is preferable. When desired, additives generally used for eye drop, such as isotonizing agent (e.g., sodium chloride), buffering agent (e.g., boric acid, disodium hydrogenphosphate, sodium dihydrogenphosphate and the like), preservative (e.g., benzalkonium chloride, benzetonium chloride, chlorobutanol and the like), tackifier [e.g., sugar (lactose, mannitol, maltose and the like), hyaluronic acid or salt thereof (sodium hyaluronate, potassium hyaluronate and the like), mucopolysaccharide (e.g., chondroitin sulfate and the like), sodium polyacrylate, carboxy vinyl polymer, crosslinked polyacrylate, and the like] may be added. The contents of the above references in this respect are hereby incorporated into the specification by reference.

The present invention is explained in more detail in the following by referring to Examples. The present invention is not limited to these examples.

### Examples

### Example 1

using FK506 as the active ingredient in the present invention, a 0.06% eye drop (suspension) having the following formulation was used as a test drug.

### Test drug

A suspension having the following formulation was produced in the same manner as in EP-A-0406791 (Example 6).

| | |
|---|---|
| FK506 | 0.6 mg |
| polyvinyl alcohol | 7.0 mg |
| disodium hydrogenphosphate 12 hydrate | 0.05 mg |
| sodium dihydrogenphosphate 2 hydrate | 0.76 mg |
| phosphoric acid | appropriate amount |
| sodium hydroxide | appropriate amount |
| sodium chloride | 8.56 mg |
| benzalkonium chloride | 0.1 mg |
| injectable water | appropriate amount |
| Total amount | 1 ml |

The above-mentioned test drug was consecutively administered twice a day for two weeks to a male (44 years old) having subjective symptoms of dry eye (sense of dryness, foreign body and grittiness) and, as a result, the subjective symptoms disappeared.

From the above result, the test drug was confirmed to be effective for the improvement of subjective symptoms of dry eye.

### Example 2

A suspension having the same formulation as in Example 1 was produced using FK506 as the active ingredient to give a 0.01% FK506 eye drop (suspension) and 0.1% FK506 eye drop (suspension) as test drugs. The base for the eye drops was used as the control drug.

The above-mentioned test drugs and the control drug were instilled four times a day for 7 days to 18 healthy subjects (6 per group) at 8:00, 11:00, 14:00 and 17:00.

The tear film breakup time (sec) of the right eye was measured before instillation and 8 days after instillation. The difference between before and after the instillation was calculated, and taken as the mean variation of the tear film breakup time.

The tear film breakup time was measured according to the conventional method. After instillation of fluorescein, the tear film was formed on the surface of the eye by nictitation. The surface of the eye was observed with a microscope without allowing nictitation, and the time until breakage of the tear film (burst by surface tension) was measured. The results are shown in Table 1.

**Table 1**

| Group | Mean variation of tear film breakup time (sec) |
|---|---|
| Control drug group | +0.17 |
| 0.01% FK506 eye drop group | +0.58 |
| 0.1% FK506 eye drop group | +0.75 |

From the above results , the test drug was confirmed to be effective for the improvement of the tear film breakup time, which is one of the tests for lacrimal fluid evaluation of dry eye.

### Industrial applicability

The use of the agent of the present invention, which comprises a macrolide compound as an active ingredient, has a superior improving effect on tear film breakup time.

This application is based on application No. 60/132,009 filed in United States of America.

## Claims

1. Use of a macrolide compound for the manufacture of a pharmaceutical agent for the treatment of dry eye by local administration to the eye, wherein the macrolide compound is a tricyclo compound of the following formula (I) 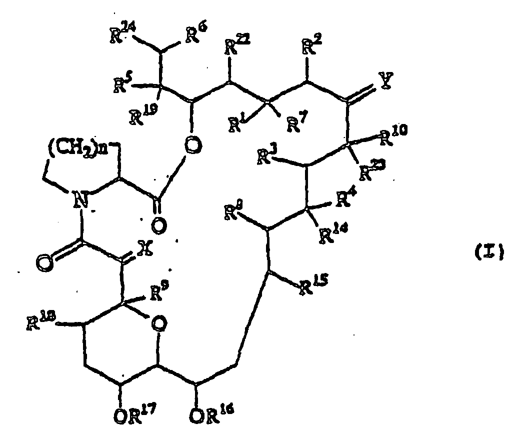 wherein
adjacent pairs of R¹ and R², R³ and R⁴, and R⁵ and R⁶ each independently
a) consist of two adjacent hydrogen atoms, wherein R² is optionally alkyl, or
b) form another bond between carbon atoms binding with the members of each pair ;
R⁷ is hydrogen atom, hydroxy, alkyloxy or protected hydroxy, or may form oxo with R¹;
R⁸ and R⁹ each independently show hydrogen atom or hydroxy ;
R¹⁰ is hydrogen atom, alkyl, alkenyl, alkyl substituted by one or more hydroxy, alkenyl substituted by one or more hydroxy, or alkyl substituted by oxo;
X is oxo, (hydrogen atom, hydroxy), (hydrogen atom, hydrogen atom), or a group of the formula -CH₂O-;
Y is oxo, (hydrogen atom, hydroxy), (hydrogen atom, hydrogen atom), or a group of the formula N-NR¹¹R¹² or N-OR¹³;
R¹¹ and R¹² each independently show hydrogen atom, alkyl, aryl or tosyl;
R¹³, R¹⁴, R¹⁵, R¹⁶, R¹⁷, R¹⁸, R¹⁹, R²² and R²³ each independently show hydrogen atom or alkyl;
R²⁴ is an optionally substituted ring which optionally contains one or more hetero atom(s); and
n is 1 or 2,
wherein
Y, R¹⁰ and R²³ optionally form, together with the carbon atom they bind with, a saturated or unsaturated 5 or 6-membered heterocyclic group containing nitrogen atom, sulfur atom and/or oxygen atom, the heterocyclic group may be substituted by one or more group (s) selected from the group consisting of alkyl, hydroxy, alkyloxy, benzyl, a group of the formula -CH₂Se(C₆H₅), and alkyl substituted by one or more hydroxy,
or a pharmaceutically acceptable salt thereof.

## Patentansprüche

1. Verwendung einer Makrolidverbindung für die Herstellung eines pharmazeutischen Mittels zur Verbesserung der Aufbrechzeit des Tränenfilms durch lokale Verabreichung auf das Auge, wobei die Makrolidverbindung eine Tricycloverbindung gemäß der folgenden Formel ( 1 ) ist: 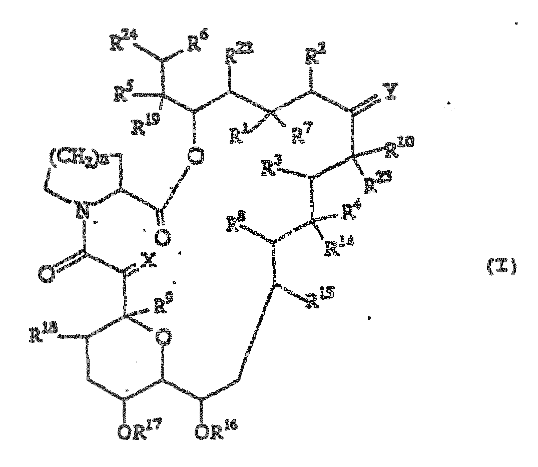 wobei
die benachbarten Paare R¹ und R², R³ und R⁴ sowie R⁵ und R⁶ jeweils unabhängig voneinander
a) aus zwei benachbarten Wasserstoffatomen bestehen, wobei R² optional Alkyl ist, oder
b) eine andere Bindung zwischen den Kohlenstoffatomen bilden, welche mit den Mitgliedern von jedem Paar verbunden sind;
R⁷ ein Wasserstoffatom, Hydroxy, Alkyloxy oder ein geschütztes Hydroxy ist, oder mit R¹ ein Oxo bilden kann;
R⁸ und R⁹ jeweils unabhängig voneinander ein Wasserstoffatom oder ein Hydroxy darstellen;
R¹⁰ ein Wasserstoffatom, Alkyl, Alkenyl, Alkyl, das mit einem oder mehreren Hydroxy substituiert ist, Alkenyl, das mit einem oder mehreren Hydroxy substituiert Ist, oder ein Alkyl ist, das mit Oxo substituiert ist;
X Oxo (Wasserstoffatom, Hydroxy), (Wasserstoffatom, Wasserstoffatom), oder eine Gruppe der Formel -CH₂O- Ist;
Y ein Oxo (Wasserstoffatom, Hydroxy), (Wasserstoffatom, Wasserstoffatom), oder eine Gruppe der Formel N-NR¹¹R¹² oder N-OR¹³ ist;
R¹¹ und R¹² jeweils unabhängig voneinander ein Wasserstoffatom, Alkyl, Aryl oder ein Tosyl sind;
R¹³, R¹⁴, R¹⁵, R¹⁶, R¹⁷, R¹⁸, R¹⁹, R²² und R²³ jeweils unabhängig voneinander ein Wasserstoffatom oder Alkyl darstellen;
R²⁴ ein optional substituierter Ring ist, der optional ein oder mehrere Heteroatome enthält; und
n 1 oder 2 ist,
wobei Y, R¹⁰ und R²³ zusammen mit dem Kohlenstoffatom, mit dem sie verbunden sind, optional eine gesättigte oder ungesättigte, 5- oder 6-gliedrige, heterocyclische Gruppe bilden, die ein Stickstoffatom, Schwefelatom und/oder Sauerstoffatom enthält, wobei die heterocyclische Gruppe mit einer oder mehreren Gruppen substituiert sein kann, die aus der Gruppe ausgewählt sind, die aus Alkyl, Hydroxy, Alkyloxy, Benzyl, eine Gruppe der Formel -CH₂Se(C₆H₅) und Alkyl, das mit einem oder mehreren Hydroxy substituiert Ist, besteht,
oder ein pharmazeutisch akzeptables Salz davon.

## Revendications

1. Utilisation d'un composé macrolide pour la fabrication d'un agent pharmaceutique pour l'amélioration du temps de rupture du film lacrymal par administration locale dans l'oeil, dans laquelle le composé macrolide est un composé tricyclo ayant la formule (I) suivante : 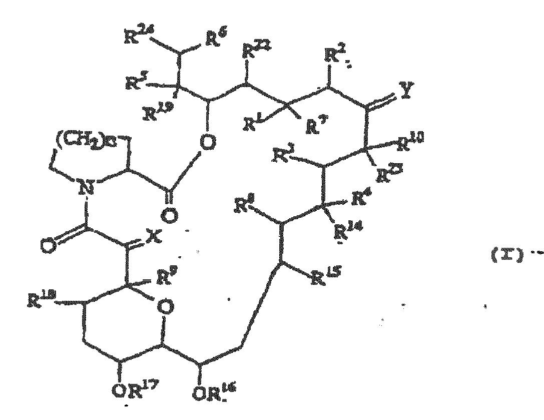 dans laquelle
des paires adjacentes de R¹ et R², R³ et R⁴, et R⁵ et R⁶, chacune indépendamment,
a) se composent de deux atomes d'hydrogène adjacents, où R² est éventuellement un groupe alkyle, ou
b) forment une autre liaison entre les atomes de carbone se liant avec les membres de chaque paire ;
R⁷ est un atome d'hydrogène, un groupe hydroxy, alkyloxy ou hydroxy protégé ou peut former un groupe oxo avec R¹ ;
R⁸ et R⁹ montrent chacun indépendamment un atome d'hydrogène ou un groupe hydroxy ;
R¹⁰ est un atome d'hydrogène, un groupe alkyle, alcényle, alkyle substitué par un ou plusieurs groupes hydroxy, alcényle substitué par un ou plusieurs groupes hydroxy, ou alkyle substitué par un groupe oxo ;
X est un groupe oxo, (atome d'hydrogène, groupe hydroxy), (atome d'hydrogène, atome d'hydrogène), ou un groupe de formule -CH₂O- ;
Y est un groupe oxo, (atome d'hydrogène, groupe hydroxy), (atome d'hydrogène, atome d'hydrogène), ou un groupe de formule N-NR¹¹R¹² ou N-OR¹³ ;
R¹¹ et R¹² montrent chacun indépendamment un atome d'hydrogène, un groupe alkyle, aryle ou tosyle ;
R¹³, R¹⁴, R¹⁵, R¹⁶, R¹⁷, R¹⁸, R¹⁹, R²² et R²³ montrent chacun indépendamment un atome d'hydrogène ou un groupe alkyle ;
R²⁴ est un cycle éventuellement substitué qui contient éventuellement un ou plusieurs hétéroatomes ; et
n vaut 1 ou 2,
dans laquelle
Y, R¹⁰ et R²³ forment éventuellement, en même temps que l'atome de carbone auquel ils se lient, un groupe hétérocyclique saturé ou insaturé à 5 ou 6 membres contenant un atome d'azote, un atome de soufre et/ou un atome d'oxygène, le groupe hétérocyclique peut être substitué par un ou plusieurs groupes choisis dans le groupe constitué par les groupes alkyle, hydroxy, alkyloxy, benzyle, un groupe de formule -CH₂Se(C₆H₅), et alkyle substitué par un ou plusieurs groupes hydroxy, ou un sel pharmaceutiquement acceptable de ceux-ci.
